# EUROPEAN PATENT SPECIFICATION

(11) **EP 0 994 353 B1**
(45) Date of publication and mention of the grant of the patent: **02.05.2003**
(21) Application number: 98203472.0
(22) Date of filing: 15.10.1998
(51) Int. Cl.: G01N 33/68, G01N 33/53

(54) **Improved method for allergy diagnostics**
Verbessertes Verfahren zur Bestimmung von Allergien
Procédé amélioré pour la détection d'allergie

(43) Date of publication of application: 19.04.2000
(73) Proprietor: Halmon Laboratoria Beheer B.V., 2011 KT Haarlem (NL)
(72) Inventor: van Ree, Ronald Dr., 1066 GN Amsterdam (NL); Aalberse, Rob C. Dr., 1115 DV Duivendrecht (NL)
(74) Representative: Büchel, Kurt F.

(56) References cited:
- EP-A- 0 051 985
- FAHLBUSCH, B. (1) ET AL: "Further characterization of IgE-binding antigens in kiwi, with particular emphasis on glycoprotein allergens." JOURNAL OF INVESTIGATIONAL ALLERGOLOGY & CLINICAL IMMUNOLOGY, (NOV.-DEC., 1998) VOL. 8, NO. 6, PP. 325-332. ISSN: 1018-9068., XP002099916
- VAN DER VEEN, MAURITS J. (1) ET AL: "Poor biologic activity of cross-reactive IgE directed to carbohydrate determinants of glycoproteins." JOURNAL OF ALLERGY AND CLINICAL IMMUNOLOGY, (1997) VOL. 100, NO. 3, PP. 327-334. ISSN: 0091-6749., XP002099917
- VAN REE, RONALD ET AL: "Lol p XI, a new major grass pollen allergen, is a member of a family of soybean trypsin inhibitor-related proteins." JOURNAL OF ALLERGY AND CLINICAL IMMUNOLOGY, (1995) VOL. 95, NO. 5 PART 1, PP. 970-978. ISSN: 0091-6749., XP002099918

## Description

### FIELD OF THE INVENTION

The present invention is in the field of allergy diagnostics and provides for an improved method to more accurately diagnose clinically relevant factors of severe and less severe allergies, particularly of food allergies. It further relates to a test kit for carrying out the diagnostic determination.

### BACKGROUND

Allergic diseases including food allergies have strongly increased over the past decades. The term "allergy" as used herein shall refer to an immunological disorder mediated by IgE antibodies, a so-called type I allergy, unless otherwise defined or derivable from the disclosure. Symptoms induced by allergenic agents or allergenic food ingredients can strongly vary with respect to severity as to the target organ that is affected. Mild symptoms are usually restricted to the oral-pharyngeal cavity: swelling of lips and tongue, itching in mouth and throat etc. This type of food allergy is often referred to as the oral allergy syndrome (OAS) (1). More severe symptoms are usually systemic and can range from generalized urticaria and gastrointestinal disorders to anaphylactic shock. In the latter case, food allergy is life threatening. Notorious examples of such dangerous food allergies are the allergies to peanuts and to shrimps. Fortunately, the mild food allergies are more common. They often occur in pollen allergic patients. A well-known example is allergy to fruits like apple and peach in patients with birch pollen allergy (2). The basis of such linkages has been shown to be crossreactivity of IgE antibodies: related structures in pollen and foods are recognized by the same IgE antibodies (2). Some of these crossreactive structures are recognized by IgE antibodies, but do not induce clinical food allergy. This has been demonstrated for IgE antibodies directed to carbohydrate determinants on glycoproteins in vegetable foods (3), including kiwi (11).

Diagnosis of food allergy is performed in several, partly complementary ways. A thorough anamnesis has to be recorded. The diagnosis can further be supported by *in vivo* measurements and *in vitro* assays. As *in vivo* technique a skin test with food allergen extracts is most common. Sometimes an oral provocation is necessary to determine which food components are the causative agents of the observed allergy. The most common *in vitro* tests are RAST and ELISA. In these assays, extracts of potentially allergic, antigenic material are immobilized to a solid phase (e.g. Sepharose, magnetic particles, polystyrene plates, etc.). After incubation of the solid phase with serum of a proband or patient, the binding of specific IgE antibodies is usually measured. For detection, radioactivity- and/or enzyme-based techniques are most frequently used. All these assays determine levels or activities of specific IgE antibodies. They do not give, however, information on the clinical appearance and relevance of the determined allergen-lgE-complexes. Similar skin test or *in vitro* test results of different patients can be accompanied by mild symptoms in one patient and by severe symptoms in another. Clinical allergy can.even be totally absent. The value of a diagnostic test would greatly improve if it would distinguish between IgE responses without clinical relevance, IgE antibodies linked to mild symptoms and IgE antibodies related to severe allergy. Such diagnostic distinction would of course considerably increase reliability and efficacy of dietary or behaviouristic recommendations. It is a major objective of the present invention to provide such a method.

The overall allergenic potency of an antigenic material may roughly be termed as the sum of allergenic potencies of its IgE-binding antigenic components. This implies that the individual IgE-profile is a crucial factor for a person's immune system to produce an allergic response against an antigenic material with which it has come into contact. It has been observed that for instance an antigenic substance A occurring e.g. in food has been recognized by an individual's immune system as an allergenic agent and causes serious symptoms, while another substance B, which may even be known to trigger allergenic reactions with many people, causes only mild symptoms or perhaps no symptoms at all to that individual. Even though the phenomena around allergy are not yet fully understood at least two factors are now known to be involved: the biochemical nature of the antigen's epitope recognized by IgE, and the chemical stability of the IgE-binding antigenic structure or determinant. If a carbohydrate side-chain on a glycoprotein is an essential part of the epitope then antigenic material, e.g. food, containing such glycoproteins is usually well tolerated (3). For induction of clinically relevant allergic symptoms, IgE recognition of antigenic epitopes of essentially peptidic nature seems to be important.

IgE-binding carbohydrate structures are extremely stable: they are insensitive to heating, proteases, etc. In contrast, several crossreactive allergenic proteins are very labile (4). This explains the poor quality of many food allergy diagnostics: clinically irrelevant antigenic carbohydrate structures retain their IgE-binding capacity, whereas relevant peptidic structures easily loose it. Some antigenic agents or components with peptidic epitopes are, however, extremely protease-resistant (5). Their IgE-binding structure is retained even after prolonged exposure to pepsin. If such a protein is insensitive to pepsin-digestion, it is more likely to be involved in severe food allergy. Pepsin-stable allergens are capable of reaching the intestines in their IgE-binding conformation. Pepsin-labile allergens are digested in the stomach, essentially limiting their allergenic reach to the oral-pharyngeal cavity. The improvements to allergy diagnostics, particularly food allergy diagnostics, provided by this invention, aim at a selective avoidance of a detection of IgE antibodies directed to clinically irrelevant carbohydrate determinants on one hand and/or at a selective detection of IgE antibodies against pepsin-stable allergens on the other hand.

### SUMMARY OF THE INVENTION

The present invention provides an improved method of allergy diagnostics to avoid or reduce the number of false positive results and/or to increase the selectivity of detection of clinically relevant allergenic factors. It thus facilitates a more accurate diagnosis, thereby increasing the quality of life and the safety of allergy, particularly food allergy, patients.

The improvement comprises avoiding the detection of carbohydrate-specific IgE antibodies and/or the selective detection of pepsin-stable allergens. Both modifications are implemented to the same type of assay: immunoassays for the measurement of allergen-specific IgE antibodies *in vitro* and *in vivo.* The present invention therefore relates to an improved method for allergy diagnostics, particularly for food allergy diagnostics, comprising reacting one or more IgE antibodies of an individual with antigenic material to be tested for allergenic potential, and subsequently determining the formation of IgE/antigen complexes, characterized in that IgE antibodies, preferably a serum sample containing IgE antibodies, obtained from said individual are exposed
a) to an agent that renders the IgE antibodies substantially incapable of reacting with a saccharidic epitope of an antigen of said antigenic material; and/or
b) to a protease-digested portion of said antigenic material; and
c) to a native, i.e., undigested portion of said antigenic material; whereupon
d) the formation of IgE/antigen-complexes is detected and evaluated with regard to the determination of unbound antigens and/or of clinically relevant IgE/antigen complexes in which the IgE antibody is bound to an essentially peptidic epitope of an antigen present in said protease-digested and/or undigested portion of the antigenic material.

The term "native" as used herein refers to undigested antigenic material, i.e., to antigenic material that has not been subjected to a proteolytic, e.g., enzymatic protease treatment.

The invention relates to a method as defined above, wherein said agent applied in step a) comprises an inhibitor that adheres or links to and/or blocks the paratope of IgE antibodies that have a specificity for antigens with saccharidic epitopes. Said agent is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract.

It is also preferred that the antigenic material be provided in the form of a food extract, a plant extract or a fruit extract, particularly in the form of a vegetable or invertebrate animal food extract, e.g., of shrimp.

Advantageously, the protein digestion of the antigenic material is carried out with at least one protease occurring in the human stomach or intestinal tract, preferably with pepsin, while the pollen extract is preferably digested using proteinase K or pronase.

The detection of IgE/antigen complexes and/or of unreacted antigens is carried out by methods well known in the art, particularly by immuno-assays such as ELISA (enzyme-linked immunosorbent assay) or RAST (radio-allergo sorbent test), or chemiluminescent methods.

In another embodiment the allergy test of the present invention is applied as a skin test wherein the IgE antibodies occurring in or released to the upper skin layers of an individual are subjected to the above-mentioned steps a) through d).

The present invention also relates to a diagnostic kit for use in one or more of the aforementioned methods, particularly for food allergy diagnostics *in vitro* or *in vivo.* Such test kits comprise
- a sample of a native, i.e., undigested antigenic material to be tested for allergenic potential; and
- an agent that renders IgE antibodies substantially incapable of reacting with a saccharidic epitope of an antigen of the antigenic material, and/or a protease-digested, preferably pepsin-digested, portion of said antigenic material.

In such test kits the aforesaid agent is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract. It is also preferred that the protease-digested antigenic material be digested by at least one protease occurring in the human stomach or intestinal tract, preferably by pepsin.

The present invention further relates to the use of a sample of a native, undigested antigenic material to be tested for allergic potential together with a protease-digested portion thereof and/or together with an inhibitor that adheres or links to and/or blocks the paratope of IgE antibodies that have a specificity for antigens with saccharidic epitopes, for the manufacture of a composition for determining an allergic response of an individual to an orally or nasally ingestible antigenic material by means of a skin test.

It is preferred that according to this use protease-digested antigenic material be used that was digested by at least one protease occurring in the human stomach or intestinal tract, preferably by pepsin.

Said agent for rendering IgE antibodies substantially incapable of reacting with essentially saccharidic epitopes of antigenic molecules is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract.

In this context the term "saccharidic epitope" as used herein shall refer to an antigenic determinant of an organic molecule, particularly of a protein or glycoprotein, wherein said determinant is substantially or completely composed of carbohydrate molecules and/or derivatives thereof.

Similarly, the term "peptidic epitope" shall refer to an antigenic determinant of an organic molecule, particularly of a protein or glycoprotein, wherein said determinant is substantially or completely composed of amino acid molecules and/or derivatives thereof, at least some of them being linked together by peptide bonding.

### BRIEF DESCRIPTION OF THE FIGURES

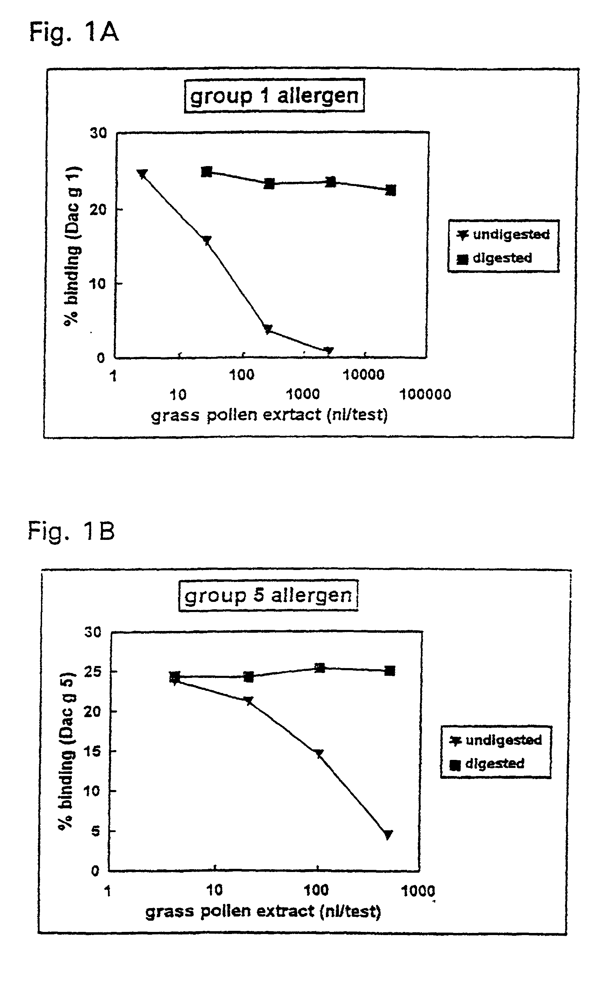

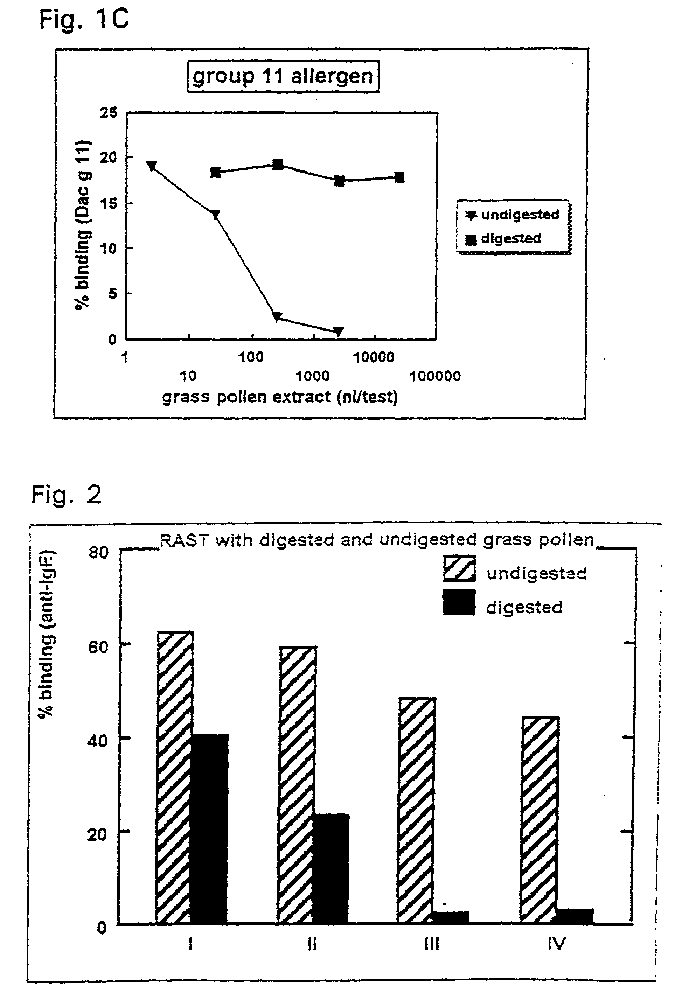

- Figs. 1A, 1B, 1C: show the influence of proteinase K digestion on the contents of three major allergens in grass pollen extract. The radiolabeled purified major allergens were subjected to a competitive RIA using digested and undigested grass pollen extract as competing agents ("inhibitors") against allergen-specific monoclonal IgE antibodies as the binding targets. The results are expressed in % binding of radiolabeled allergen.
- Fig. 2: displays a RAST with undigested and proteinase K digested grass pollen extract with 4 sera of grass pollen allergic patients. Serum I and II clearly recognize antigens still present in digested pollen extract, although there is a significant decrease compared to undigested pollen extract. This difference is caused by a loss of peptidic epitopes. Serum Ill and IV do not bind to digested pollen extract, indicating that reactivity of the IgE antibodies of those sera with pollen extract is exclusively directed to peptidic epitopes.

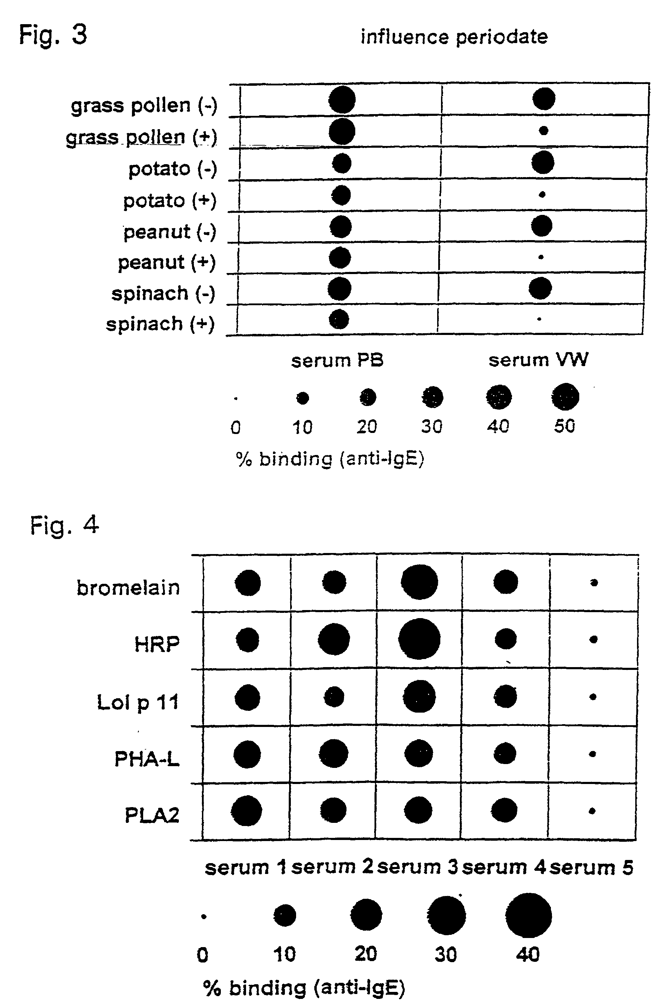

- Fig. 3: demonstrates the influence of periodate treatment of pollen and food extracts in a RAST with untreated (-) and treated (+) extracts. For serum PB, periodate treatment did not have a significant effect on IgE binding. For serum VW, IgE binding almost completely disappeared after periodate treatment indicating that this serum contains IgE antibodies primarily reactive with periodate-sensitive carbohydrate determinants.
- Fig. 4: discloses binding of carbohydrate-specific IgE to unrelated glycoproteins. A RAST was performed with 4 sera that contain IgE antibodies that are reactive with protease-digested pollen extracts. One serum of a pollen-allergic patient (serum 5) was included that did not show IgE reactivity with digested pollen extracts. Five totally unrelated glycoproteins were tested: bromelain from pineapple stem, horseradish peroxidase (HRP), Lol p 11 from *Lolium perenne* grass pollen, phytohemagglutinin-L (PHA-L) from *Phaseolus vulgaris*, and phospholipase A₂ (PLA₂) from bee venom. The 4 carbohydrate-reactive sera bind to all 5 glycoproteins, the control serum is unreactive. The explanation for this observation is that these unrelated proteins and antigens present in pollen extract have very similar carbohydrate structures in their IgE-binding epitopes.

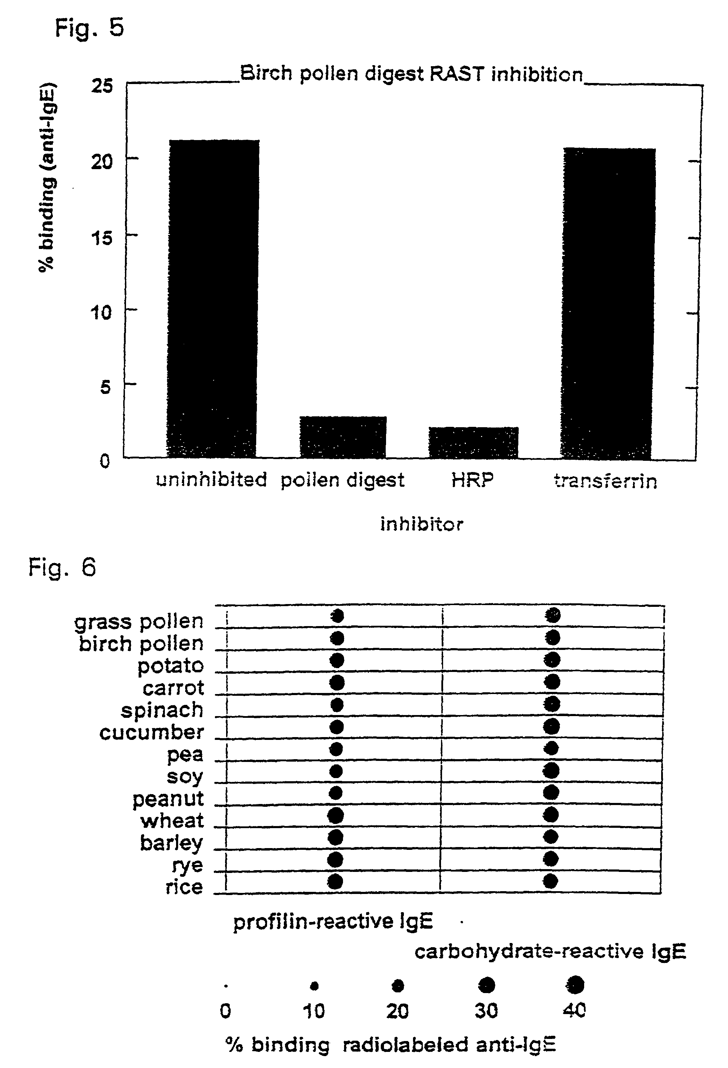

- Fig. 5: discloses how much the binding activity of IgE antibodies to Sepharose-coupled protease-digested pollen (in this case birch pollen) extract is inhibited by the addition of protease-digested birch pollen extract, horseradish peroxidase (HRP) and the human glycoprotein transferrin. The latter protein was used as a control glycoprotein with a N-glycan that is different from the typical plant N-glycans. Both digested pollen extract and HRP inhibited the binding almost completely. With transferrin, however, no inhibition was observed.
- Fig. 6: displays the results obtained when a serum containing IgE antibodies against carbohydrate determinants, and another serum containing IgE antibodies against profilin were tested in a RAST with different pollen extracts and vegetable foods. On the basis of these RAST results alone it is practically impossible to distinguish between these two types of sera.

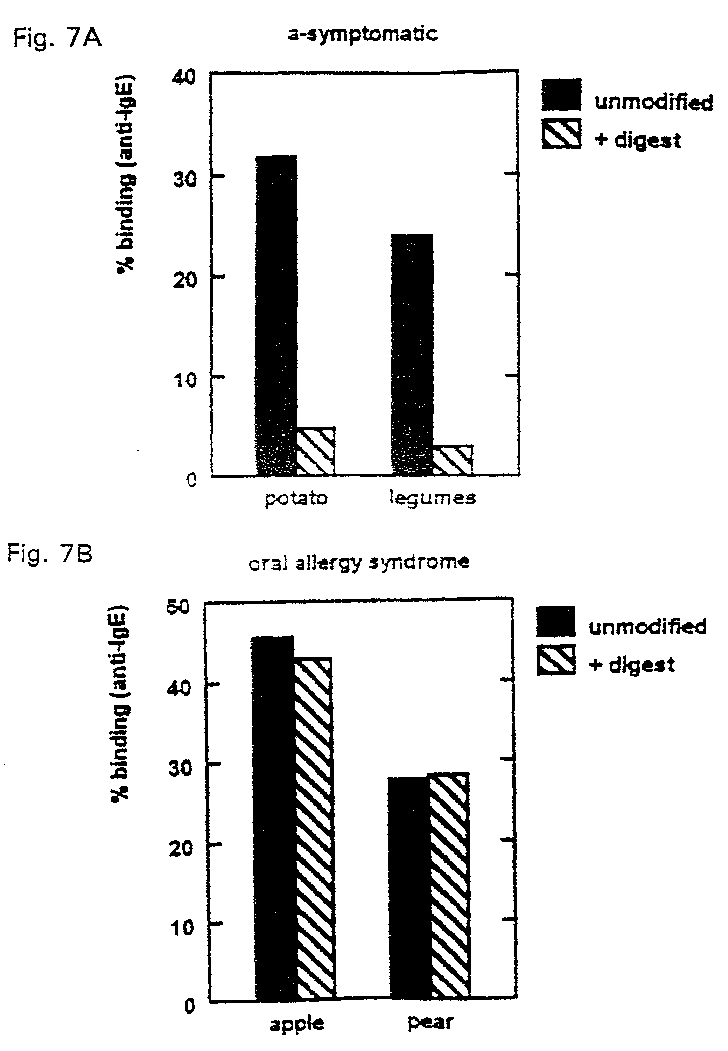

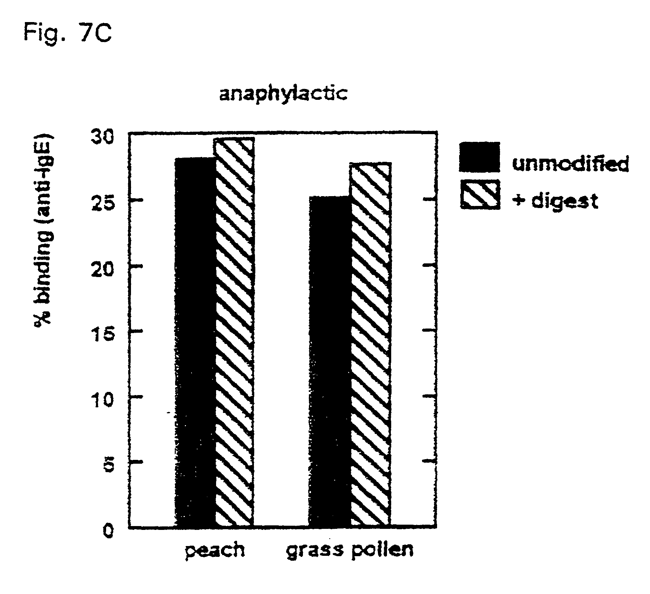

- Figs. 7A, 7B, 7C: show the results of a modified RAST to avoid the detection of clinically irrelevant IgE antibodies against carbohydrate determinants. A comparison was made between a conventional (unmodified) RAST and the modified RAST (i.e., in the presence of protease-digested pollen extract) according to the present invention. For this purpose serum of three typical patients was used: 1) a pollen-allergic patient with a positive RAST to vegetable foods without any related symptoms of food allergy (asymptomatic); 2) a birch pollen-allergic patient with mild symptoms of food allergy (oral allergy syndrome), based on pollen-vegetable food crossreactive allergens; 3) a grass pollen-allergic patient with more severe fruit allergy (anaphylaxis), but no crossreactivity between pollen and vegetable food. It is demonstrated that the conventional RAST yields a false positive result for the asymptomatic patient whereas the modified RAST yields a much more realistic picture on the potential allergic threat.

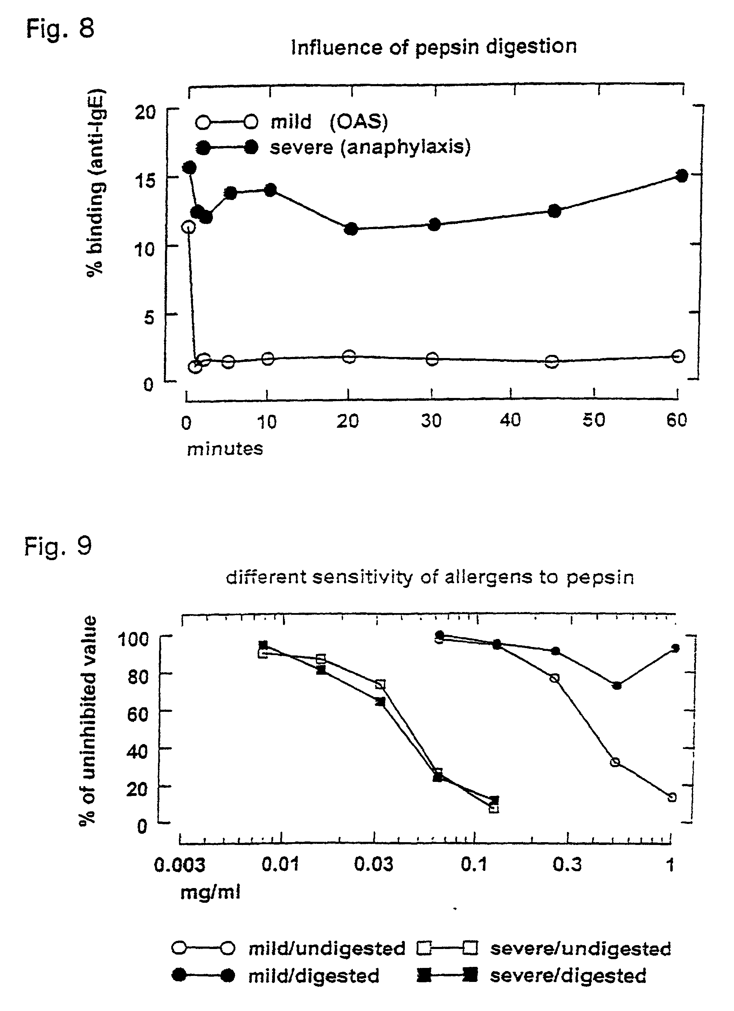

- Fig. 8: shows RAST results using a pepsin-treated peach extract as an allergen source. The peach extract was digested by pepsin for different periods of time (1 min to 1 hr). After digestion, samples of the extract were coupled to Sepharose for application in a RAST. The RAST analysis was performed with serum from a patient with a birch pollen-related mild fruit allergy (OAS) and with a serum from a patient suffering from a more severe fruit allergy (anaphylaxis). While the IgE antibodies of both patients are reactive with undigested peach extract, virtually no IgE reactivity of patient 1 (OAS) was observed with pepsin-digested peach extract. This effect occurred already with peach extract that has been pepsin-treated for just 1 minute. For the other patient the RAST value did not significantly change, not even when using a peach extract that has been subjected to a 1 hour pepsin treatment. This indicates that the second patient's IgE antibodies recognize an allergen that is extremely resistant to pepsin treatment. In other words, they recognize and bind to an allergenic molecule that will very likely pass the stomach in an IgE-binding conformation.
- Fig. 9: relates to a RAST inhibition with undigested and pepsin-treated (for 1 hour) peach extract. Serum of a patient with a mild allergy (OAS) and another serum from a patient with severe allergy (anaphylaxis were used. After pepsin digestion, the inhibitory potency of the extract did not change for the serum of the patient with severe allergy, which indicates that the patient's IgE antibodies selectively recognize and bind to an epitope that is not destroyed by pepsin treatment. For the serum of the patient with OAS a decrease of at least one order of magnitude was observed indicating that the patient's IgE antibodies link to a peptidic epitope that is present in undigested peach extract but that is absent in digested peach extract due to cleavage by pepsin.

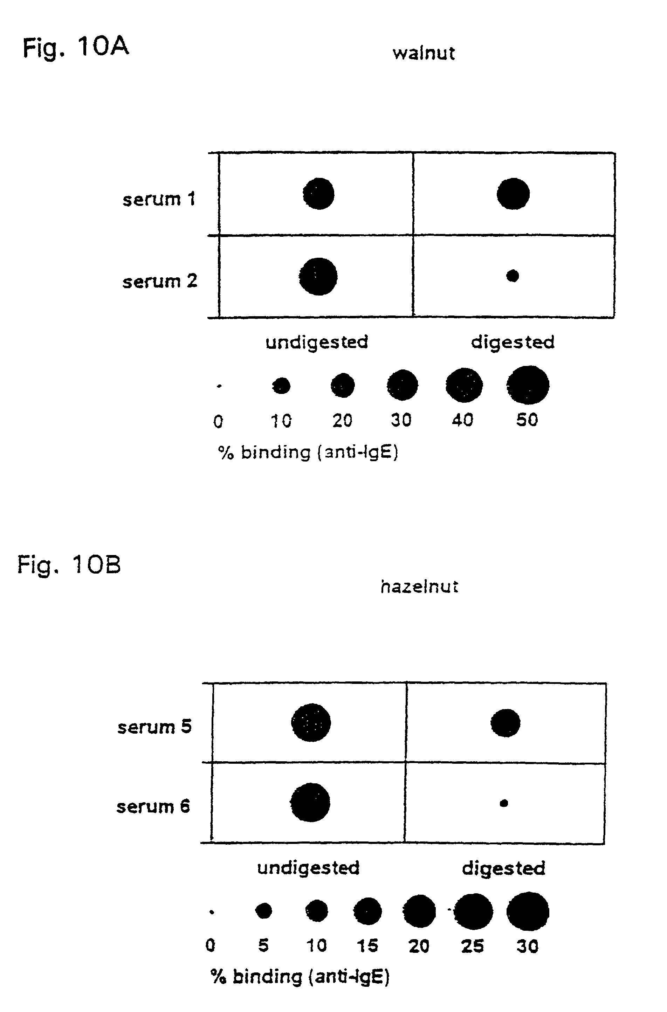

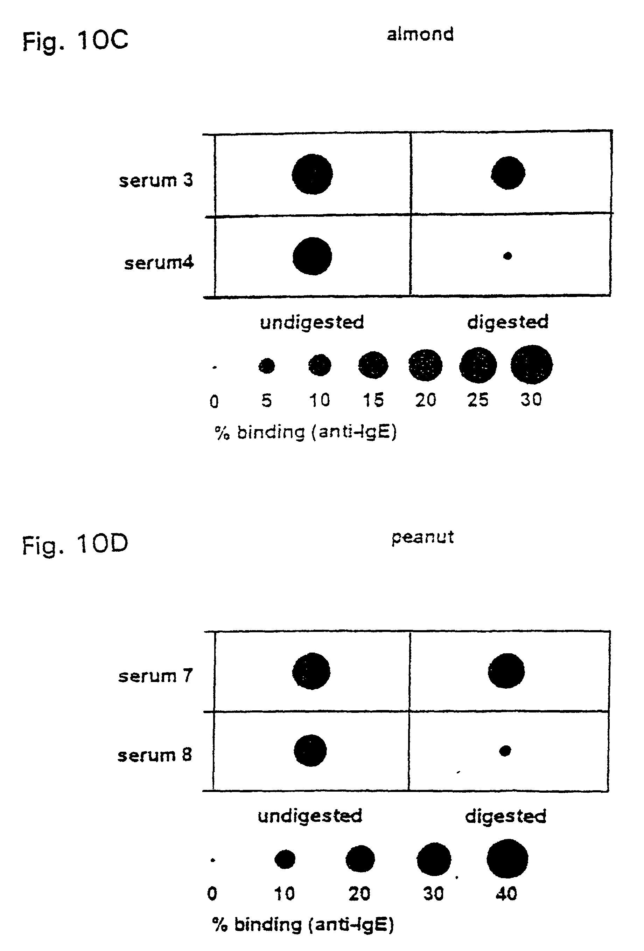

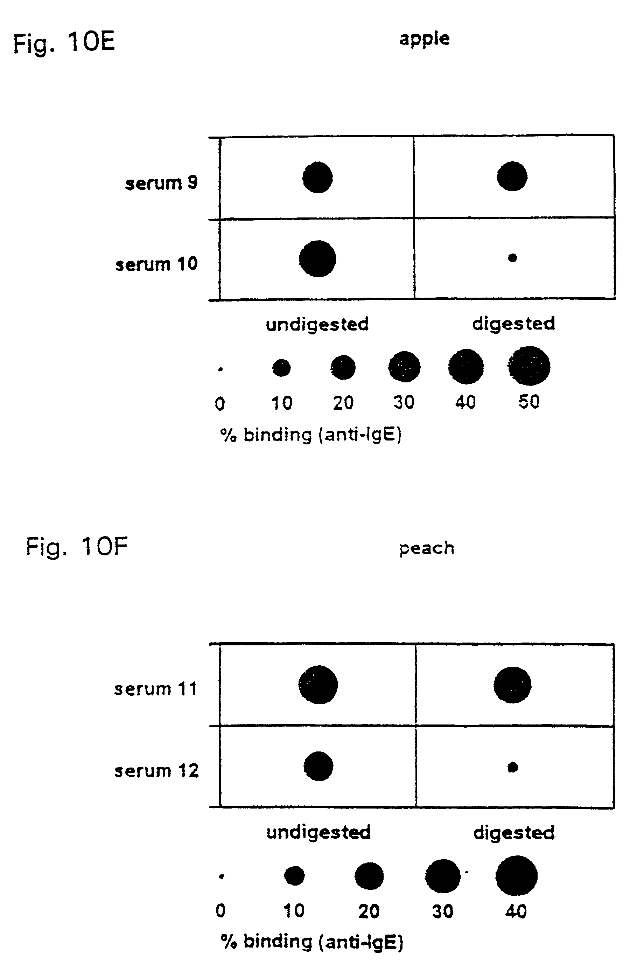

- Figs. 10A, 10B, 10C, 10D, 10E, 10F: display results obtained from RAST analysis of various native (i.e. undigested) and pepsin-digested food extracts. For several kinds of nut and fruit the reactivity of a serum containing IgE antibodies against pepsin-insensitive and a serum containing IgE antibodies against pepsin-sensitive allergen(s) has been investigated. The modified RAST according to the present invention clearly distinguishes between both types of sera.

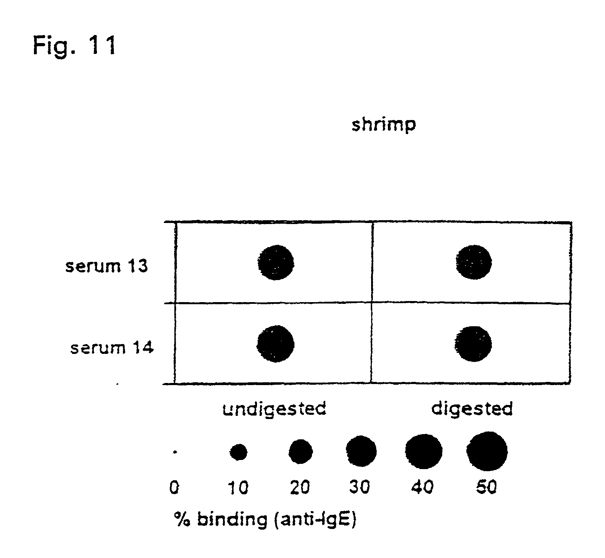

- Fig. 11: shows the result of a RAST with undigested and pepsin-digested shrimp allergen extract. For two sera of patients suffering from severe allergy related to shrimp it is demonstrated that the IgE-binding structures are insensitive to digestion by pepsin.

### DETAILED DESCRIPTION OF THE INVENTION

Assays for the measurement of specific IgE have the following characteristics. The desired antigenic material, e.g., a supposed or known allergenic agent such as a food extract, is immobilized on a solid phase, either by covalent binding to a chemically-activated solid phase (e.g. Sepharose, magnetic beads, paper discs etc.) or by non-covalent coating (e.g. to polystyrene microwell plates). Serum obtained from an individual, usually from an allergic or potentially allergic patient, is incubated with the solid phase to allow allergen-specific IgE antibodies to bind to the immobilized allergens. In some assays, the antigenic material is haptenized (e.g. biotinylated) and incubated with serum, prior to the addition of a solid phase that contains ligands (e.g. streptavidin) with affinity for the hapten. In this case, the immobilization of the antigens occurs after the formation of specific IgE-antigen-complexes. All assays have in common that the incubation of serum and antigenic material is followed by several washing steps to remove non-bound serum components. Subsequently, a second incubation is carried out with antibodies directed to human IgE. These antibodies are labeled to allow detection of bound antibodies after incubation. Labeling can be achieved in many ways like with radioactivity, with enzymes (e.g. horseradish peroxidase or alkaline phosphatase) or with fluorescent or chemiluminescent labels. The second incubation is again followed by several washing steps to remove non-bound labeled anti-IgE. Detection of bound label is performed with the appropriate techniques well known in the art.

To avoid the detection of carbohydrate-specific IgE antibodies with food allergen diagnostics, a soluble inhibitor is added in the first incubation step (serum plus antigen). The soluble inhibitor reacts with and blocks the carbohydrate-specific IgE antibodies, thereby preventing them from binding to the solid phase. This soluble inhibitor can be a protease-digested extract of pollen. Protease-digestion by e.g. proteinase K or pronase results in destruction of peptide backbones of (glyco)proteins, but leaves carbohydrate structures untouched (6). Pollen are the prime choice as a source for saccharidic epitopes, because induction of carbohydrate-specific IgE antibodies primarily occurs by inhalation of pollen (2). These IgE antibodies are crossreactive to closely related carbohydrate structures in vegetable food. Therefore, protease-digested vegetable food extracts may serve as alternative inhibitors. Finally, synthetic peptides mimicking saccharidic epitopes can also be used (7,8).

To selectively detect IgE antibodies directed to allergens that survive passage through the stomach and thus may cause severe allergies, the antigenic material is subjected to pepsin digestion. In the case of freeze-dried bulk food extract (that is normally used in food allergy diagnostics) as the antigenic material the extract is subjected to a 1 hour digestion by pepsin at 37°C. Pepsin can either be soluble or immobilized to a solid matrix like agarose. Digestion is carried out in 10 mMol/L HCl at protein concentrations (allergen extract) of about 1 to 5 mg/ml and pepsin concentrations of 0.35% (w/v). After 1 hour, the digestion is stopped by addition of a 1/2 volume of 0.5 Mol/L K₂HPO₄ (pH 8.2). The digestion is followed by a dialysis against a suitable medium for further use of the digested allergen extract (for instance, coupling to a solid phase). According to the best mode of the invention the selective detection of IgE antibodies directed to stable, i.e., pepsin-resistant IgE epitopes of peptidic nature is combined with the addition of an IgE inhibitor that allows to avoid detection of IgE antibodies directed to similarly stable but clinically irrelevant saccharidic epitopes.

In conventional skin tests the antigenic material, e.g., a food extract, is injected into the skin in order to measure the IgE-induced immediate allergic response. This response is mediated by crosslinking of mast cell bound IgE antibodies. This requires recognition of at least two epitopes on a single allergen molecule. In protease-digested pollen extract the saccharidic epitopes are free monovalent IgE-binding entities. They will, therefore, not act as a crosslinking agent but block mast cell- bound IgE antibodies with carbohydrate specificity. Any possible skin reaction mediated by these IgE antibodies will therefore be inhibited if protease-digested pollen extract is added to the skin test material.

When using pepsin-digested antigenic material only pepsin-insensitive allergens will be capable of inducing a skin reaction. Skin test materials treated with pepsin are, therefore, suitable reagents for testing *in vivo* IgE responses to stable allergens, including stable food allergens, that enter an individual's body via the oral route.

In order that the invention described herein may be more fully understood, the following examples are set forth. The examples are for illustrative purposes only and are not to be construed as limiting this invention in any respect. In particular, the person skilled in the art will understand that the results reported hereinafter for food allergens may well be extrapolated to any other orally ingested antigenic material of similar or comparable chemical nature.

### Example 1: Reduction or prevention of detection of false positive results

### A Protease-digestion of pollen extract

Pollen extracts were digested with proteinase K or pronase by an overnight incubation at 50°C. For digestion of 25 mg of pollen proteins approximately 3 units of proteinase K or pronase, respectively, were used following a method described in (6). It is essential to check whether the peptide backbone of (allergenic) proteins has been sufficiently digested in order to make sure that virtually all (>99.9%) of the peptidic epitopes have been destroyed, i.e., cleaved or otherwise rendered inactive. For this purpose the contents of three single major allergens, namely group 1 allergen (Fig. 1A), group 5 allergen (Fig. 1B) and group 11 allergen (Fig. 1C), were measured with monoclonal IgE antibody-based immunoassays. Figures 1A, 1B and 1C illustrate the high degree of protein digestion of these three major allergens in proteinase K treated grass pollen extract, as determined in competitive RIA's: when using proteinase K-digested grass pollen extract as the competing agent ("inhibitor") the concentrations of the IgE-bound purified radiolabeled major allergens remain virtually unaffected, i.e., constant at the starting levels, even at the highest concentrations of inhibitor applied. On the other hand, when using undigested pollen extract, the native allergens contained therein compete with the radiolabeld purified allergens for binding to the allergen-specific IgE antibodies, resulting in a dose-dependent inhibition and thus in a decrease of detectable IgE-bound radiolabled allergens. These results indicate that in all three cases at least 99% of the original activity, i.e., of the original IgE-binding peptidic epitopes of the tested major allergens, were destroyed. As a second control for effective digestion, undigested and digested extracts were analyzed by SDS-PAGE in conjunction with silverstaining. The digested extracts virtually lacked all protein bands originally present in the extracts.

### B Proof of existence of IgE antibodies directed to saccharidic epitopes

Protease-digested grass pollen extract was coupled to Sepharose for application in a RAST. The amount of digest coupled to Sepharose was equivalent to the amount of undigested grass pollen extract (4mg dry-weight on 100 mg Sepharose) coupled thereto for routine testing. Fig. 2 illustrates that some sera recognized digested and undigested pollen extract, whereas others only recognized undigested pollen extract. This was a first indication that the former group might have recognized saccharidic epitopes. Fig. 3 shows that IgE-binding was lost after periodate treatment of allergen extracts, indicating the involvement of sugar determinants. Furthermore, sera that were active in IgE binding to digested pollen extracts also recognized totally unrelated purified glycoproteins with which they had only a similar N-glycan in common (Fig.4). The RAST to a digested pollen extract sample was completely inhibited by such an unrelated glycoprotein (Fig.5). Together, these data demonstrate that a positive RAST for protease-digested pollen extracts is explained by IgE-binding to carbohydrate determinants.

### C Degree of crossreactivity

Sera of patients with carbohydrate-specific IgE antibodies were shown to have a positive RAST to a broad spectrum of vegetable foods (2). By RAST-inhibition these IgE antibodies were shown to be highly crossreactive. However, not all sera with such a broad spectrum of IgE crossreactivity have anti-carbohydrate antibodies. In some cases IgE antibodies to the highly crossreactive allergen profilin were shown to be involved (1,2,9). It is very hard to distinguish between both types of sera on the basis of current food RAST's (Fig.6). Reactivity with profilin, however, is recognized to indicate a high likelihood to develop clinical food allergy in many cases, whereas subjects with anti-carbohydrate IgE lack food-related allergy symptoms.

### D Poor biologic activity of IgE against saccharidic epitopes

A group of grass pollen allergic patients (n = 173) was screened in a RAST for IgE antibodies against peanut (3). Almost 1/3 (n = 50) had a clear positive peanut RAST. None of these patients, however, developed clinical allergy symptoms related to peanut. When tested for IgE antibodies against carbohydrate structures, more than 90% were positive, suggesting that antibodies with this specificity were largely responsible for the false-positive peanut RAST results. This was confirmed in a peanut RAST-inhibition assay. The peanut RAST was completely inhibited by digested grass pollen extract. This was not the case for sera of patients with a clinical peanut allergy. These data imply that IgE antibodies against carbohydrate determinants are poor inducers, if at all, of a release of mediator molecules that initiate an allergic reaction and that are released from effector cells, mainly mast cells and basophils. This was confirmed both *in vivo* and *in vitro.*

Skin prick tests with peanut extract are negative in non-symptomatic subjects. Histamine-release from basophils of non-symptomatic individuals can be induced but only at peanut extract concentrations approximately 1000-fold higher than those required for a corresponding stimulation of basophils of symptomatic individuals. This finding confirms that carbohydrate-specific IgE antibodies have poor biologic activity and detection of such antibodies is not indicative of a likelihood to develop a clinical food allergy.

### E Improved method

As pointed out, the improved method aims at avoiding the detection of carbohydrate-specific IgE antibodies in diagnostic tests for food allergy. This can be achieved by addition of soluble protease-digested pollen extract. At a concentration of 50 µg per test the false positive peanut RAST were completely inhibited, whereas clinically relevant positive tests stayed positive (3). This approach was also tested for other vegetable foods. In Fig. 7A two examples are shown of the inhibition of a false-positive RAST for vegetable foods. As a control, sera taken from patients suffering from clinical allergy symptoms were tested using the conventional and the improved RAST methods (Fig. 7B, Fig. 7C). It was observed that the exposure of these sera to protease-digested pollen extract according to the present invention did not result in an inhibition of the IgE antibodies which confirms the assumption that in these cases the IgE antibodies are directed to the clinically more relevant peptidic epitopes of the tested food allergens.
It was important to realize that IgE reactivity with vegetable food is not necessarily either completely anti-carbohydrate or anti-peptide. Sera can and usually do contain mixed populations of IgE antibodies having specificities against different antigenic components of vegetable food. This means that inhibition of a RAST with protease-digested pollen extracts can also be partial. The aim of the improved method according to the present invention is to reduce or totally prevent the detection of IgE antibodies that are directed to saccharidic epitopes of antigenic molecules.

### Example 2: Improving accuracy and reliability of positive results

### A Pepsin digestion of food allergen extracts

As mentioned above the clinical appearance of allergy to food ingredients, particularly of vegetable food, can differ quite considerably. Birch pollen allergic patients with allergy to fruits like apple and peach usually demonstrate mild local symptoms (OAS). The basis for the coincidence of inhalant allergy and food allergy is found in the presence of related allergens in pollen and fruit (2). Crossreactive IgE antibodies directed to the birch pollen allergen Bet v 1 and to the pollen allergen profilin recognize homologous counterparts in fruits. Patients with more severe symptoms upon consumption of fruits like apple and peach do not necessarily have pollen allergy (10). The allergy to fruits in this case is independent from a sensitization to pollen.

Allergens in fruit that cause more severe forms of food allergy are extremely resistant to digestion by pepsin. The typical crossreactive allergens related to pollen are completely digested within minutes. The stability was tested by using samples of a peach extract, exposed for different times to pepsin, in a RAST with serum samples from a mild (pollen-crossreactive) and a severe (non-pollen reactive) fruit allergic patient (Fig.8). Serum of these patients was also used in a peach RAST inhibition with pepsin-digested and undigested peach extract. After 1 hour of pepsin digestion, only the RAST of the patient with more severe symptoms was inhibited (Fig.9).

### B Improved method

To selectively measure IgE antibodies directed to pepsin-insensitive allergens, food extracts were treated with pepsin prior to immobilization on Sepharose. This approach was tested with several food allergen extracts: apple, peach, hazelnut, walnut, peanut and almond. In addition, the approach was also tested for food extracts of animal origin known to cause severe food allergies, particularly for extracts obtained from invertebrate animals such as shrimp. The approach was to test different types of patients with mild pollen-related food allergy and with more severe "real" food allergy and to find out whether pepsin-digestion would result in a selective detection of IgE antibodies in the latter group (i.e. those with more severe allergy symptoms).

In Figures 10A through 10F it is illustrated that the approach indeed distinguishes between both types of patients. Some sera have a positive RAST with digested and undigested, others only with undigested extracts. We also tested several sera obtained from patients with known allergy to shrimp (which is an important representative of invertebrate animals being part of many dishes and food creations) using both digested and undigested extracts. Here we did not find any significant decrease in IgE binding after pepsin treatment (Fig.11). This confirms the stability and potential allergenicity of food allergens occurring in shrimp and probably in related invertebrates alike.

### REFERENCES

1. van Ree. The oral allergy syndrome. In: Contact urticaria syndrome. Eds. S. Amin, A. Lahti, H.I. Maibach. CRC Press, Boca Raton. 1997;289-299.
2. van Ree, R.C. Aalberse. Pollen vegetable food crossreactivity; serological and clinical relevance of crossreactive IgE. J. Clin. Immunoassay 1993; 16:124-130.
3. van der Veen, R. van Ree, R.C. Aalberse, J.H. Akkerdaas, S.J. Koppelman, H.M. Jansen, J.S. van der Zee. Poor biologic activity of cross-reactive IgE directed to carbohydrate determinants of glycoproteins. J. Allergy Clin. Immunol. 1997;100:327-334.
4. A. Jankiewicz, H. Aulepp, W. Baltes, K.W. Bögl, L.I. Dehne, T. Zuberbier, S. Vieths. Allergic sensitization to native and heated celery root in pollen-sensitive patients investigated by skin test and IgE binding. Int. Arch. Allergy Immunol. 1996;111:268-278.
5. J.D. Astwood, J.N. Leach, R.L. Fuchs. Stability of food allergens to digestion in vitro. Nature Biotechnol. 1996;14:1269-1273
6. R. van Ree, D. Hoffman, W. van Dijk, V. Brodard, K. Mahieu, C.A.M. Koeleman, M. Grande, W.A. van Leeuwen, R.C. Aalberse, Lol p 11, a new major grass pollen allergen, is a member of a family of soybean trypsin inhibitor-related proteins. J. Allergy Clin. Immunol. 1995;95:970-978.
7. K.R. Oldenburg, D. Loganathan, I.J. Goldstein, P.G. Schultz, M.A. Gallop. Peptide ligands for a sugar-binding protein isolated from a random peptide library. Proc. Natl. Acad. Sci. USA. 1992;89:5393-5397.
8. S.L. Harris, L. Craig, J.S. Mehroke, M. Rashed, M.B. Zwick, K. Kenar, E.J. Toone, N. Greenspan, F.-I. Auzanneau, J.-R. Marino-Albernas, B.M. Pinto, J.K. Scott. Exploring the basis of peptide-carbohydrate crossreactivity: evidence for discrimination by peptides between closely realted anti-carbohydrate antibodies. Proc. Natl. Aca. Sci. USA. 1997;94:2454-2459.
9. R. van Ree, V. Voitenko, W.A. van Leeuwen, R.C. Aalberse. Profilin is a cross-reactive allergen in pollen and vegetable foods. Int. Arch. Allergy Immunol. 1992;98:96-104.
10.M. Fernandez Rivas, R. van Ree, M. Cuevas. Allergy to *Rosaceae* fruits without related pollinosis. J. Allergy Clin. Immunol. 1998; in press.
11. B. Fahlbush et al (1998) Invest. Allergol. Clin. Immunol. 8, 325-332.

## Claims

1. A method for allergy diagnostics, particularly for food allergy diagnostics, comprising reacting one or more IgE antibodies of an individual with antigenic material to be tested for allergenic potential, and subsequently determining the formation of IgE/antigen complexes, **characterized in that** IgE antibodies, preferably a serum sample containing IgE antibodies, obtained from said individual are exposed to
a) an inhibitor that adheres or links to and/or blocks the paratope of IgE antibodies that have a specificity for antigens with saccharidic epitopes, wherein the inhibitor is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract, thereby rendering the IgE antibodies substantially incapable of reacting with a saccharidic epitope of an antigen of said antigenic material; and/or to
b) a protease-digested portion of said antigenic material; and to
c) an undigested portion of said antigenic material; whereupon the formation of IgE/antigen-complexes is detected and evaluated with regard to the determination of clinically relevant IgE/antigen complexes in which the IgE antibody is bound to an essentially peptidic epitope of an antigen occurring in said protease-digested and/or in said undigested portion of said antigenic material.

2. The method according to claim 1, **characterized in that** the antigenic material is a food extract, a plant extract or a fruit extract, particularly a vegetable or animal food extract.

3. The method according to any one of claims 1 or 2, **characterized in that** the protein digestion of the antigenic material is carried out with at least one protease occurring in the human stomach or intestinal tract, preferably with pepsin.

4. The method according to claim 1 **characterized in that** the inhibitor is a pollen or vegetable food extract digested by proteinase K or pronase.

5. The method according to any one of claims 1 to 4, **characterized in that** the detection of IgE/antigen complexes and/or of unbound antigens is carried out by immuno-assays such as ELISA (enzyme-linked immunosorbent assay) or RAST (radio-allergo sorbent test), or by chemiluminescent methods.

6. A diagnostic kit that comprises
a) a sample of a native, undigested antigenic material to be tested for allergenic potential; and
b) an inhibitor that adheres or links to and/or blocks the paratope of IgE antibodies that have a specificity for antigens with a saccharidic epitope and which renders IgE antibodies substantially incapable of reacting with saccharidic epitopes present in a protease-digested and/or undigested portion of said antigenic material, wherein the inhibitor is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract; and/or
c) a protease-digested, preferably pepsin-digested, portion of said antigenic material, which allows an estimation of the expected severity of the allergic response or a selective detection of IgE antibodies in patients with more severe allergy symptoms;
for use in a method according to any one of claims 1 to 5.

7. Use of a sample of a native, undigested antigenic material to be tested for allergic potential together with a protease-digested portion thereof and/or together with an inhibitor that adheres or links to and/or blocks the paratope of IgE antibodies that have a specificity for antigens with saccharidic epitopes, wherein the inhibitor is selected from the group consisting of a protease-digested pollen extract, a protease-digested vegetable food extract, a peptide that mimics the structure or antigenic nature of a glycan, and a synthetic molecule comprising a saccharidic epitope of essentially the same composition and structure as occurring in a pollen extract or in a vegetable food extract, for the manufacture of a composition for determining an allergic response of an individual to an orally or nasally ingestible antigen material, which is to be applied in a skin test.

8. Use according to claim 7 , wherein the protease-digested antigenic material is digested by at least one protease occurring in the human stomach or intestinal tract, preferably by pepsin.

## Patentansprüche

1. Verfahren zur Bestimmung von Allergien, insbesondere für die Bestimmung von Nahrungsmittelallergien, darin bestehend, einen oder mehr IgE-Antikörper eines Individuums mit Antigenmaterial reagieren zu lassen, das auf sein allergenisches Potential geprüft werden soll, und danach die Bildung von IgE-Antigen-Komplexen zu bestimmen, **dadurch gekennzeichnet, dass** von dem benannten Individuum gewonnene IgE-Antikörper, bevorzugt ein IgE-Antikörper enthaltendes Serummuster, ausgesetzt werden:
a) einem Inhibitor, der an das Paratop von IgE-Antikörpern, die eine Spezifität für Antigene mit saccharidischen Epitopen haben, anhaftet, sich daran bindet und/oder es blockiert, wobei der Inhibitor aus der Gruppe ausgewählt ist, die aus einem mit Protease aufgeschlossenen Pollenextrakt, einem mit Protease aufgeschlossenen Extrakt planzlicher Nahrung, einem Peptid, das die Struktur oder antigene Natur eines Glykans nachahmt, und einem synthetischen Molekül, das ein saccharidisches Epitop von im Wesentlichen der gleichen Zusammensetzung und Struktur, wie sie in einem Pollenextrakt oder einem Extrakt pflanzlicher Nahrung auftreten, besteht, wodurch die IgE-Antikörper im Wesentlichen unfähig gemacht werden, mit einem saccharidischen Epitop eines Antigens dieses Antigenmaterials zu reagieren; und/oder
b) einem mit Protease aufgeschlossenen Anteil dieses Antigenmaterials; und
c) einem unaufgeschlossenen Anteil dieses Antigenmaterials;
woraufhin die Bildung von IgE-Antigen-Komplexen nachgewiesen und bezüglich der Bestimmung von klinisch relevanten IgE-Antigen-Komplexen, in denen der IgE-Antikörper an ein im wesentlichen peptidisches Epitop eines Antigens gebunden ist, das in diesem mit Protease aufgeschlossenen und/oder diesem unaufgeschlossenen Anteil des Antigenmaterials vorkommt, bewertet wird.

2. Verfahren gemäss Anspruch 1, **dadurch gekennzeichnet, dass** das Antigenmaterial ein Nahrungsmittelextrakt, ein Pflanzenextrakt oder ein Fruchtextrakt ist, insbesondere ein Extrakt pflanzlicher oder tierischer Nahrungsmittel.

3. Verfahren nach Anspruch 1 oder 2, **dadurch gekennzeichnet, dass** der Proteinaufschluss des Antigenmaterials mit zumindest einer Protease ausgeführt wird, die im menschlichen Magen oder Darmkanal auftritt, bevorzugt mit Pepsin.

4. Verfahren nach Anspruch 1, **dadurch gekennzeichnet, dass** der Inhibitor ein durch Proteinase K oder Pronase aufgeschlossener Pollenextrakt oder Extrakt eines pflanzlichen Nahrungsmittels ist.

5. Verfahren nach einem der Ansprüche 1 bis 4, **dadurch gekennzeichnet, dass** der Nachweis von IgE-Antigen-Komplexen und/oder von ungebundenen Antigenen durch Immunoassays wie ELISA (*enzyme-linked immunosorbent assay*) oder RAST (*radio-allergo sorbent test*) oder durch Chemilumineszenzverfahren ausgeführt wird.

6. Diagnosekit, umfassend:
a) ein Muster von nativem, unaufgeschlossenem Antigenmaterial, das auf sein allergenes Potential getestet werden soll; und
b) einen Inhibitor, der an das Paratop von IgE-Antikörpern, die eine Spezifität für Antigene mit einem saccharidischen Epitopen haben, anhaftet, sich daran bindet und/oder es blockiert und der IgE-Antikörper im Wesentlichen unfähig macht, mit saccharidischen Epitopen zu reagieren, die in einem mit Protease aufgeschlossenen und/oder unaufgeschlossenen Anteil dieses Antigenmaterials vorliegen, wobei der Inhibitor aus der Gruppe ausgewählt ist, die aus einem mit Protease aufgeschlossenen Pollenextrakt, einem mit Protease aufgeschlossenen Extrakt planzlicher Nahrung, einem Peptid, das die Struktur oder antigene Natur eines Glykans nachahmt, und einem synthetischen Molekül, das ein saccharidisches Epitop von im Wesentlichen der gleichen Zusammensetzung und Struktur, wie sie in einem Pollenextrakt oder einem Extrakt pflanzlicher Nahrung auftreten, besteht; und/oder
c) einen mit Protease aufgeschlossenen, bevorzugt einen mit Pepsin aufgeschlossenen Anteil dieses Antigenmaterials, was eine Abschätzung der erwarteten Heftigkeit der allergischen Reaktion oder einen selektiven Nachweis von IgE-Antikörpern in Patienten mit schwereren Allergiesymptomen erlaubt;
zur Verwendung in einem Verfahren nach einem der Ansprüche 1 bis 5.

7. Verwendung eines Musters von nativem, unaufgeschlossenem Antigenmaterial, das auf allergisches Potential geprüft werden soll, zusammen mit einem mit Protease aufgeschlossenen Anteil davon und/oder zusammen mit einem Inhibitor, der an das Paratop von IgE-Antikörpern, die eine Spezifität für Antigene mit saccharidischen Epitopen haben, anhaftet, sich daran bindet und/oder es blockiert und der aus der Gruppe ausgewählt ist, die aus einem mit Protease aufgeschlossenen Pollenextrakt, einem mit Protease aufgeschlossenen Extrakt planzlicher Nahrung, einem Peptid, das die Struktur oder antigene Natur eines Glykans nachahmt, und einem synthetischen Molekül, das ein saccharidisches Epitop von im Wesentlichen der gleichen Zusammensetzung und Struktur, wie sie in einem Pollenextrakt oder einem Extrakt pflanzlicher Nahrung auftreten, besteht, zur Herstellung einer Zusammensetzung für die Bestimmung einer Allergiereaktion in einem Individuum auf ein Antigenmaterial, das oral oder nasal aufgenommen werden kann und das auf einen Hauttest angewendet werden soll.

8. Verwendung gemäss Anspruch 7, worin das mit Protease aufgeschlossene Antigenmaterial durch zumindest eine Protease aufgeschlossen wird, die im menschlichen Magen oder Darmkanal vorkommt, bevorzugt durch Pepsin.

## Revendications

1. Procédé destiné à un diagnostic d'allergies, en particulier à un diagnostic d'allergies alimentaires, comprenant la mise en réaction d'un ou plusieurs anticorps IgE d'un individu avec une substance antigénique à tester en ce qui concerne un potentiel allergénique, puis la détermination de la formation de complexes IgE/antigènes, **caractérisé en ce que** les anticorps IgE, de préférence un échantillon de sérum contenant des anticorps IgE, obtenus à partir dudit individu sont exposés à
a) un inhibiteur qui adhère ou se lie au paratope d'anticorps IgE et/ou bloque le paratope d'anticorps IgE qui présentent une spécificité pour les antigènes comportant des épitopes de type saccharide, où l'inhibiteur est sélectionné à partir du groupe constitué d'un extrait de pollen digéré par une protéase, d'un extrait alimentaire végétal digéré par une protéase, d'un peptide qui imite la structure ou la nature antigénique d'un glycanne, et d'une molécule synthétique comprenant un épitope de type saccharide de sensiblement la même composition et la même structure que celles qui apparaissent dans un extrait de pollen ou dans un extrait alimentaire végétal, en rendant ainsi les anticorps IgE pratiquement incapables de réagir avec un épitope de type saccharide d'un antigène de ladite substance antigénique, et/ou à
b) une partie digérée par une protéase de ladite substance antigénique, et à
c) une partie non digérée de ladite substance antigénique, après quoi la formation de complexes IgE/antigène est détectée et évaluée en ce qui concerne la détermination de complexes IgE/antigène cliniquement pertinents dans lesquels l'anticorps IgE est lié à un épitope essentiellement peptidique d'un antigène apparaissant dans ladite partie digérée par une protéase et/ou ladite partie non digérée de ladite substance antigénique.

2. Procédé selon la revendication 1, **caractérisé en ce que** la substance antigénique est un extrait alimentaire, un extrait de plante ou un extrait de fruit, en particulier un extrait alimentaire végétal ou animal.

3. Procédé selon l'une quelconque de revendications 1 ou 2, **caractérisé en ce que** la digestion de protéines de la substance antigénique est exécutée par l'apparition d'au moins une protéase dans l'estomac ou les voies intestinales chez l'humain, de préférence avec une pepsine.

4. Procédé selon la revendication 1 **caractérisé en ce que** l'inhibiteur est un extrait de pollen ou alimentaire végétal digéré par une protéinase K ou une pronase.

5. Procédé selon l'une quelconque de revendications 1 à 4, **caractérisé en ce que** la détection de complexes IgE/antigènes et/ou d'antigènes non liés est exécutée par des essais immunologiques tels qu'un essai ELISA (dosage par immunosorbant lié à une enzyme) ou un essai RAST (essai radioallergologique par allergosorbant) ou par des procédés de chimioluminescence.

6. Nécessaire pour diagnostique qui comprend
a) un échantillon d'une substance antigénique non digérée, native à tester en ce qui concerne un potentiel allergénique, et
b) un inhibiteur qui adhère ou se lie au paratope d'anticorps IgE et/ou bloque le paratope d'anticorps IgE qui présentent une spécificité pour des antigènes présentant un épitope de type saccharide et qui rend les anticorps IgE sensiblement incapables de réagir avec des épitopes de type saccharide présents dans une partie digérée par une protéase et/ou une partie non digérée de ladite substance antigénique, où l'inhibiteur est sélectionné à partir du groupe constitué d'un extrait de pollen digéré par une protéase, un extrait alimentaire végétal digéré par une protéase, d'un peptide qui imite la structure ou la nature antigénique d'un glycanne, et d'une molécule synthétique comprenant un épitope de type saccharide de composition et de structure presque identiques à celles apparaissant dans un extrait de pollen ou un extrait alimentaire végétal, et/ou
c) une partie digérée par une protéase, de préférence une partie digérée par une pepsine de ladite substance antigénique, qui permet une estimation de la gravité prévue de la réponse allergique ou une détection sélective d'anticorps IgE chez des patients présentant des symptômes d'allergies plus importants, en vue d'une utilisation dans un procédé selon l'une quelconque de revendications 1 à 5.

7. Utilisation d'un échantillon d'une substance antigénique non digéré, natif devant être testé en ce qui concerne un potentiel allergique en association avec une partie digérée par une protéase de celui-ci et/ou en association avec un inhibiteur qui adhère ou se lie au paratope d'anticorps IgE et/ou bloque le paratope d'anticorps IgE qui présentent une spécificité pour des antigènes présentant des épitopes de type saccharides, où l'inhibiteur est sélectionné à partir du groupe constitué d'un extrait de pollen digéré par une protéase, d'un extrait alimentaire végétal digéré par une protéase, d'un peptide qui imite la structure ou la nature antigénique d'un glycanne, et d'une molécule synthétique comprenant un épitope de type saccharide présentant presque la même composition et la même structure que celles qui apparaissent dans un extrait de pollen ou dans un extrait alimentaire végétal, afin de fabriquer une composition destinée à déterminer une réponse allergique d'un individu à une substance antigénique pouvant être ingérée par voie buccale ou par voie nasale, qui doit être appliquée lors d'un test cutané.

8. Utilisation selon la revendication 7, dans laquelle ladite substance antigénique digérée par une protéase est digérée par au moins une protéase apparaissant dans l'estomac ou les voies intestinales chez l'humain, de préférence par une pepsine.
